Europäisches Patentamt

European Patent Office

Office européen des brevets

(19)

(11) Veröffentlichungsnummer : **0 035 307
B1**

(12) **EUROPÄISCHE PATENTSCHRIFT**

(45) Veröffentlichungstag der Patentschrift :
22.06.83

(21) Anmeldenummer : 81200214.5

(22) Anmeldetag : 24.02.81

(51) Int. Cl.³ : **A 61 B 6/14, A 61 C 19/04**

(54) **Dental-Tomographiegerät.**

(30) Priorität : **01.03.80 DE 3007935**

(43) Veröffentlichungstag der Anmeldung :
**09.09.81 Patentblatt 81/36**

(45) Bekanntmachung des Hinweises auf die Patenterteilung : **22.06.83 Patentblatt 83/25**

(84) Benannte Vertragsstaaten :
**DE FR GB NL SE**

(56) Entgegenhaltungen :
**FR A 531 720
GB A 1 463 198
US A 3 239 935
US A 3 536 079
US A 4 039 837**

(73) Patentinhaber : **Philips Patentverwaltung GmbH
Steindamm 94
D-2000 Hamburg 1 (DE)
DE
N.V. Philips' Gloeilampenfabrieken
Groenewoudseweg 1
NL-5621 BA Eindhoven (NL)
FR GB NL SE**

(72) Erfinder : **Bergman, Gerard P. M.
Fleminglaan 13
Eindhoven (NL)**
Erfinder : **Schreiber, Peter
Königsberger Strasse 20
D-2084 Rellingen (DE)**
Erfinder : **Steenfadt, Eberhard
Bremervörder Strasse 73
D-2160 Stade (DE)**

(74) Vertreter : **Hartmann, Heinrich, Dipl.-Ing. et al
Philips Patentverwaltung GmbH Steindamm 94
D-2000 Hamburg 1 (DE)**

Jouve, 18, rue St-Denis, 75001 Paris, France

# 0 035 307

« Dental-Tomographiegerät »

Die Erfindung betrifft ein Dental-Tomographiegerät mit einem um eine senkrechte Achse schwenkbaren Träger, an dem eine Strahlenquelle und ein darauf ausgerichteter Filmhalter befestigt sind, mit einer Verstelleinrichtung zur translatorischen Verschiebung des Trägers in einer horizontalen Ebene, mit Antriebsmotoren zum Schwenken des Trägers, zum translatorischen Verschieben des Trägers und zum Verschieben eines Films innerhalb des Filmhalters, und mit einer digitalen Steuereinrichtung zur Steuerung der Antriebsmotoren.

Ein solches Dental-Tomographiegerät ist aus der DE-B-26 30 135 bekannt. Durch die digitale Steuereinrichtung werden die beiden Motoren zur translatorischen Verschiebung des Trägers in der Horizontalebene so gesteuert, dass die Schwenkachse des Trägers auf einer Kurve verläuft, die wenigstens annähernd der Gebissform entspricht. Der Antriebsmotor für den Träger schwenkt den Träger dabei jeweils so, dass die Verbindungslinie zwischen dem Brennfleck der Strahlenquelle und einer am Filmhalter vorgesehenen Spaltblende, durch die hindurch die Strahlung auf dem Film auftrifft, ungefähr senkrecht zu der Kurve verläuft. Der Film wird innerhalb des Filmhalters mit einer Geschwindigkeit verschoben, die proportional zur und grösser als die Translationsgeschwindigkeit des Trägers ist.

Die Schärfe der mit einem solchen Gerät hergestellten Panoramaaufnahme des Zahnbogens hängt davon ab, wie genau die kurve, auf der der Träger translatorisch verschoben wird, mit der Gebissform übereinstimmt. Zu diesem Zweck enthält die Steuereinrichtung mehrere Steuerprogramme für jeweils eine Verstellkurve des Trägers, von denen eines ausgewählt werden kann. Die Auswahl kann z. B. dadurch erfolgen, dass von dem aufzunehmenden Gebiss ein Gipsabdruck angefertigt wird, der mit den verschiedenen Verstellkurven verglichen wird, wobei die Kurve ausgewählt wird, die mit der Gebissform am meisten übereinstimmt.

Auch wenn dabei eine grosse Anzahl von Steuerprogrammen entsprechend einer grossen Anzahl verschiedener Gebissformen gespeichert ist, können sich dabei aus verschiedenen Gründen noch Ungenauigkeiten und damit Unschärfen sowie Verzerrungen (weil sich die Vergrösserung während der Aufnahme ändert) der Schichtaufnahme ergeben. Beispielsweise kann versehentlich eine Verstellkurve gewählt werden, die mit der Gebissform relativ ungenau übereinstimmt. Es kann vorkommen, dass bei der Aufnahme der Patient seinen Kopf so ausgerichtet hat, dass die Symmetrieebene seines Gebisses nicht mit der Symmetrieebene bzw. -linie der vorgewählten Verstellkurve übereinstimmt. Es kann auch vorkommen, dass der Patient seinen Kopf so hält, dass die Zahnspitzen des Oberkiefers nicht in der Horizontalebene liegen, die der Brennfleck bei der Schichtaufnahme durchfährt.

Aufgabe der vorliegenden Erfindung ist es, ein Dental-Tomographiegerät der eingangs genannten Art so auszugestalten, dass sich schärfere, verzerrungsfreie Schichtaufnahmen ergeben.

Diese Aufgabe wird erfindungsgemäss dadurch gelöst, dass die digitale Steuereinrichtung einen mit beim Zubeissen betätigbaren Druckkontakten versehenen, in den Patientenmund einführbaren Körper und einen Rechner enthält, der aus dem Schaltzustand der einzelnen Druckkontakte Steuersignale errechnet, die zur Steuerung der Antriebsmotoren für eine Verschiebung der Achse auf einer die zubeißende Gebißform und -Länge annähernden Kurve dienen.

Zur Anfertigung einer Schichtaufnahme wird also der mit Druckkontakten versehene Körper in den Mund des Patienten in eine vorbestimmte Position eingeführt. Der Patient beisst zu, wodurch der Teil der Druckkontakte, der zwischen den Zahnspitzen des Ober- bzw. Unterkiefers liegt, betätigt wird. Der Schaltzustand der einzelnen Druckkontakte (betätigt bzw. nicht betätigt) ist daher ein kennzeichen der jeweiligen Gebissform und Gebisslage. Dieser Schaltzustand wird vom Rechner abgefragt. Nachdem der Kopf des Patienten durch Kopfstützen fixiert ist, kann gegebenenfalls der Körper durch eine geeignete Beißsstütze ersetzt werden. Der Rechner errechnet aus dem Schaltzustand die jeweilige Gebissform bzw. eine Kurve, die die Gebissform sehr genau annähert, und liefert entsprechende Steuersignale für die Antriebsmotoren.

Eine Weiterbildung der Erfindung sieht vor, dass der Körper eine Platte umfaßt, auf der durch Zubeissen zusammendrückbare Kontakte angeordnet sind. Dadurch wird berücksichtigt, dass die Zahnspitzen eines Gebisses im allgemeinen nicht exakt in einer Ebene liegen, was bei Druckkontakten, die bei ihrer Betätigung ihre Form nicht verändern, zu Schwierigkeiten führen würde, weil möglicherweise einige Kontakte, die unmittelbar unter bzw. über eine Zahnspitze liegen, nicht betätigt werden würden. Bei den zusammendrückbaren Kontakten besteht diese Gefahr nicht, weil beim Zubeissen alle Kontakte betätigt werden, die zwischen den Zähnen liegen — wenn auch nicht gleichzeitig.

Eine besonders geeignete Ausführungsform des Körpers besteht nach einer Weiterbildung der Erfindung darin, dass auf der Grundplatte eine Vielzahl von Noppen aus einem Material mit niedrigem Elastizitätsmodul angeordnet ist, von dem ein Teil eine geringere Höhe hat als der restliche Teil, und auf der von der Grundplatte abgewandten Stirnseite mit einem elektrisch leitfähigen Belag versehen ist, und dass die Noppen von einer elastischen Membran überspannt sind, die auf ihrer den Noppen zugewandten Seite mit einer leitfähigen Schicht versehen ist. Unter einem Material mit niedrigem Elastizitätsmodul wird hier ein Material verstanden, das es gestattet, mit relativ niedriger Gebisskraft die Noppen um einige Millimeter herunterzudrücken — beispielsweise Schaumgummi oder ein ähnlicher Stoff. Beim Zusammenbeissen werden zunächst die Membran und die höheren Noppen zusammengedrückt, bis die leitende Schicht auf der Membran mit der leitenden Schicht auf der Stirnseite der kürzeren Noppen, die mit jeweils einer Signalleitung verbunden ist, in Kontakt kommt. Die Membran mit der leitfähigen Schicht und die mit einem elektrisch leitfähigen Belag versehenen Stirnflächen der Noppen bilden also Druckkontakte, d. h. eine elektrische Verbindung, deren Widerstandswert in Abhängigkeit von dem darauf einwirkenden Druck um Zehner-Potenzen änderbar ist. Die höheren, nicht mit einem elektrisch leitfähigen Belag versehenen Noppen, von denen jeweils mindestens vier eine Noppe mit elektrisch leitendem Belag umgeben, sorgen dafür, dass die Membran im unbelasteten Zustand nicht die mit einem elektrischen Belag versehenen Noppen berühren kann.

Die Intensität der auf den Film auftreffenden Strahlung hängt erheblich von der jeweiligen Schwenkstellung des Trägers ab. Befindet sich der Träger beispielsweise in einer Stellung, in der die Vorderzähne abgebildet werden, dann wird zugleich auch die Wirbelsäule durchstrahlt, wodurch die Strahlung erheblich geschwächt wird. Dies kann dazu führen, dass dieser Bereich auf dem Film schwächer belichtet wird als die übrigen Teile, wenn die translatorische Verschiebung in der horizontalen Ebene mit einer dem Betrage nach konstanten Geschwindigkeit erfolgt. Eine andere weiterbildung der Erfindung sieht daher vor, dass eine Dosisleistungsmesseinrichtung vorgesehen ist, deren Ausgangssignal dem Rechner zwecks Steuerung der Geschwindigkeit der Antriebsmotoren entsprechend der jeweils gemessenen Dosisleistung zugeführt wird.

Die Erfindung wird nachstehend anhand eines in der Zeichnung dargestellten Ausführungsbeispiels näher erläutert. Es zeigen :

Figur 1   die geometrischen Verhältnisse bei einem Dental-Tomographiegerät,
Figur 2a den mit Druckkontakten versehenen Körper in einer Seitenansicht und
Figur 2b den Körper in der Draufsicht,
Figur 3   eine Seitenansicht des Gerätes,
Figur 4   eine Draufsicht auf das Gerät,
Figur 5   eine Schaltung zur Steuerung der Antriebsmotoren mit einem Microcomputer,
Figur 6   den Aufbau des Microcomputers und
Figur 7   bis 11 Flussdiagramme, in den en der Steuerungsablauf des Dental-Tomographiegerätes nach der Erfindung wiedergegeben wird.

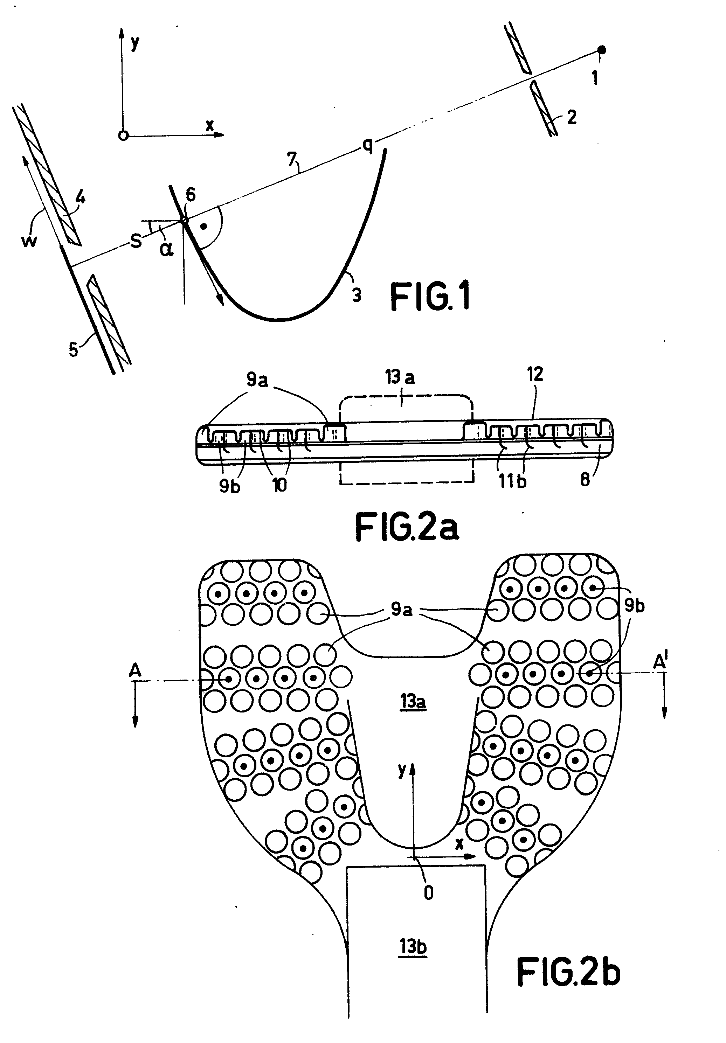

In Fig. 1 ist mit 1 eine Strahlenquelle, z. B. eine Röntgenröhre, bezeichnet, deren Strahlung durch eine Spaltblende 2 mit vertikal angeordnetem Spalt hindurchtritt, das Gebiss, dessen Form durch die Kurve 3 dargestellt ist, durchquert und durch eine mit einem nicht näher dargestellten Filmhalter verbundene Spaltblende 4 hindurch, deren Spalt ebenfalls senkrecht verläuft, auf einen in dem Filmhalter angeordneten Film 5 trifft. Die Strahlenquelle 1 und die Spaltblende 2 sowie der nicht näher dargestellte Filmhalter und die mit ihm verbundene Spaltblende 4 sind starr an einem Träger befestigt, dessen Richtung durch die Gerade 7 angedeutet ist, und der um eine Achse 6 schwenkbar ist. Der Abstand der Achse 6 von der Strahlenquelle ist mit q und der Achsensabstand von der Filmebene ist mit s bezeichnet. Zur Anfertigung einer Schichtaufnahme muss der Träger in einer horizontalen Ebene so verschoben werden, dass seine Schwenkachse 6 eine mit dem Zahnbogen 3 zusammenfallende Bahn beschreibt. Dabei muss gleichzeitig der Träger um die Achse 6 so geschwenkt werden, dass die Strahlung den Zahnbogen etwa senkrecht durchsetzt. Gleichzeitig muss der Film 5 in der entgegengesetzten Richtung mit einer Geschwindigkeit bewegt werden, die der Geschwindigkeit der Schwenkachse 6 proportional ist, wobei der Proportionalitätsfaktor der Vergrösserung entspricht, mit der das Gebiss auf dem Film abgebildet wird.

Die Vergrösserung m berechnet sich nach der Gleichung :

$$m = (q + s)/s \qquad (1)$$

Dann gilt für die Geschwindigkeit w des Films 5 innerhalb des Filmhalters :

$$w = m \cdot v \qquad (2)$$

wobei v die Geschwindigkeit ist, mit der sich die Schwenkachse 6 entlang der Kurve bewegt.

Der Wert der Geschwindigkeit v hat auf die Schärfe der Schichtaufnahme keinen Einfluss, jedoch auf die Belichtung. Eine gleichmässig belichtete Schichtaufnahme des Gebisses ergibt sich nämlich nur, wenn die Geschwindigkeit v der Dosisleistung D(t) proportional ist, die je nach Stellung des Trägers schwanken kann. Wenn beispielsweise die Vorderzähne des Patienten durchstrahlt werden, muss zugleich auch dessen wirbelsäule durchstrahlt werden, so dass die Intensität erheblich abnimmt. Die Verschiebung muss daher im Bereich der Vorderzähne langsamer erfolgen. Mithin gilt für die Geschwindigkeit v :

$$v = c \cdot D(t) \qquad (3)$$

wobei c eine Konstante ist, die von der Geometrie der Anordnung und von der Empfindlichkeit des Filmes abhängt. Der Momentanwert der Dosisleistung kann mittels einer vor oder hinter dem Film aufgestellten Dosisleistungsmesseinrichtung, z. B. einer Ionisationskammer, erfasst werden. Die Verschiebung der Schwenkachse in der Zeichenebene muss im allgemeinen mit zwei voneinander unabhängigen Antriebsmotoren zur Verschiebung in zwei zueinander senkrechten Richtungen erfolgen, z. B. in der in der Zeichnung angegebenen y- und x-Richtung. Die Geschwindigkeitskomponenten in diesen Richtungen errechnen sich dann nach :

$$v = \sqrt{(dx/dt)^2 + (dy/dt)^2} = c \cdot D(t) \qquad (4)$$

Die Geschwindigkeit dy/dt des Antriebs in y-Richtung ergibt sich dann aus der Geschwindigkeit dx/dt nach der Gleichung :

$$dv/dt = dy/dx \cdot dx/dt = y' \cdot dx/dt \qquad (5)$$

wobei y' die erste Ableitung von y nach x ist. Aus den Gleichungen (4) und (5) ergibt sich :

$$dx/dt = c \cdot D(t)/\sqrt{1 + (y')^2} \qquad (6)$$

Aus den Gleichungen (5) und (6) ergibt sich die Geschwindigkeit in y-Richtung :

$$dy/dt = y' \cdot c \cdot D(t)/\sqrt{1 + (y')^2} \qquad (7)$$

Für den Winkel α, den der Träger dabei mit der x-Achse einschliessen muss, gilt :

$$\alpha = arc\ ctg(- y') \qquad (8)$$

Die Werte α, dx/dt, dy/dt und w lassen sich nur berechnen, wen y' bekannt ist. Dazu muss wiederum y bekannt sein, d. h. eine analytische Funktion, deren Verlauf der Form des Gebissbogens entspricht. Um diese Funktion ermitteln zu können, ist der in Fig. 2a und 2b dargestellte Körper vorgesehen, wobei Fig. 2a einen Querschnitt längs der Linie a − a' in Fig. 2b darstellt und wobei der Körper etwa im Massstab 2 : 1 vergrössert dargestellt ist.

Der Körper umfasst eine ebene Grundplatte 8, die mit einer Vielzahl zylinderförmiger Noppen aus Schaumgummi versehen ist. Dabei gibt es eine erste Gruppe von etwas höheren Noppen 9a und eine zweite Gruppe von Noppen, die etwas niedriger, z. B. 1/2 mm, sind als die anderen Noppen und auf ihrer Stirnfläche mit einem leitfähigen Belag versehen sind, die mit je einer elektrischen Anschlussleitung 11b verbunden sind, die im Innern der Platte geführt oder durch die Platte hindurch geführt sind. Jeder der niedrigeren Noppen 9b, die in Fig. 2b mit einem Punkt versehen sind und die im folgenden als Schaltnoppen bezeichnet werden, ist von wenigstens vier höheren Noppen 9a umgeben.

Die Noppen werden von einer gummiartigen Membran 12 überspannt, die auf ihrer den Noppen zugewandten Seite mit einer elektrisch leitenden Schicht versehen ist. Die Schaltnoppen 9b bilden also zusammen mit der Membran 12 Schalt- bzw. Druckkontakte, die normalerweise geöffnet sind und durch einen Druck auf die Membran oberhalb der betreffenden Schaltnoppe geschlossen werden. Wie aus Fig. 2b ersichtlich, sind insgesamt 30 Schaltnoppen vorhanden, deren Lage aus der nachfolgenden Tabelle hervorgeht :

| x (cm) | y (cm) | x (cm) | y (cm) | x (cm) | y (cm) | x (cm) | y (cm) |
|---|---|---|---|---|---|---|---|
| + 1,0 | 0,75 | + 1,20 | 1,60 | + 1,4 | 2,7 | + 1,5 | 4,0 |
| − 1,0 | 0,75 | − 1,20 | 1,60 | − 1,4 | 2,7 | − 1,5 | 4,0 |
| + 1,28 | 0,47 | + 1,59 | 1,52 | + 1,8 | 2,7 | + 1,9 | 4,0 |
| − 1,28 | 0,47 | − 1,59 | 1,52 | − 1,8 | 2,7 | − 1,9 | 4,0 |
| + 1,57 | 0,18 | + 1,98 | 1,44 | + 2,2 | 2,7 | + 2,3 | 4,0 |
| − 1,57 | 0,18 | − 1,98 | 1,44 | − 2,2 | 2,7 | − 2,3 | 4,0 |
| | | + 2,38 | 1,37 | + 2,6 | 2,7 | + 2,7 | 4,0 |
| | | − 2,38 | 1,37 | − 2,6 | 2,7 | − 2,7 | 4,0 |

Dabei ist die Lage der y-Achse durch die Symmetrielinie des Körpers gegeben, und die x-Achse liegt wie in Fig. 2b eingezeichnet. Um die Vorderzähne des Gebisses in eine wenigstens annähernd definierte Position bezüglich des Körpers zu bekommen, ist die Grundplatte 8 mit über die Membran 12 hinausragenden Erhöhungen 13a und 13b versehen, so dass sich nach dem Zubeissen die Schneidezähne zwischen den beiden Erhöhungen (ungefähr im Koordinatenursprung) befinden.

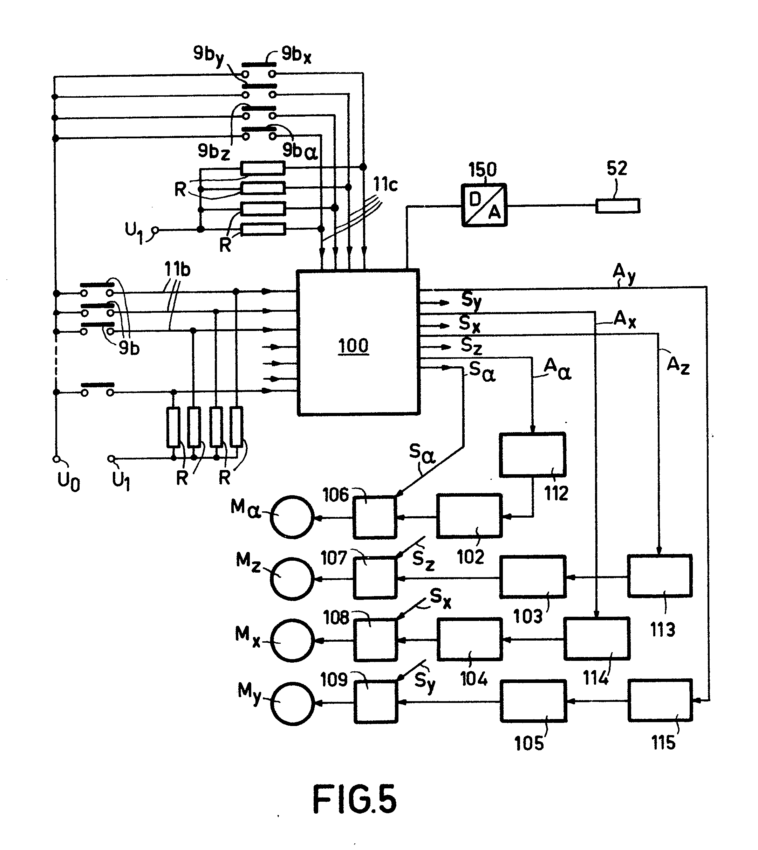

Beim Zubeissen wird von jeder der vier bzw. drei jeweils in einer Linie nebeneinander liegenden Schaltnoppen mindestens eine betätigt, gegebenenfalls aber auch zwei oder drei. Wird nur eine betätigt, so werden in dem noch in Verbindung mit Fig. 5 zu beschreibenden Rechner die Koordinaten dieser Schaltnoppe zur Kennzeichnung des Zahnbogens gespeichert. Werden zwei Schaltnoppen betätigt, so wird der Mittelwert ihrer Koordinaten gebildet. Wenn drei Schaltnoppen betätigt werden, werden die Koordinaten der mittleren Schaltnoppe zur Kennzeichnung der Lage des Zahnbogens gespeichert. Der Schaltzustand der Schaltnoppen ist daher ein Kennzeichen für die Form des Gebisses und seine Lage in bezug auf die mit Noppen versehene Platte 8.

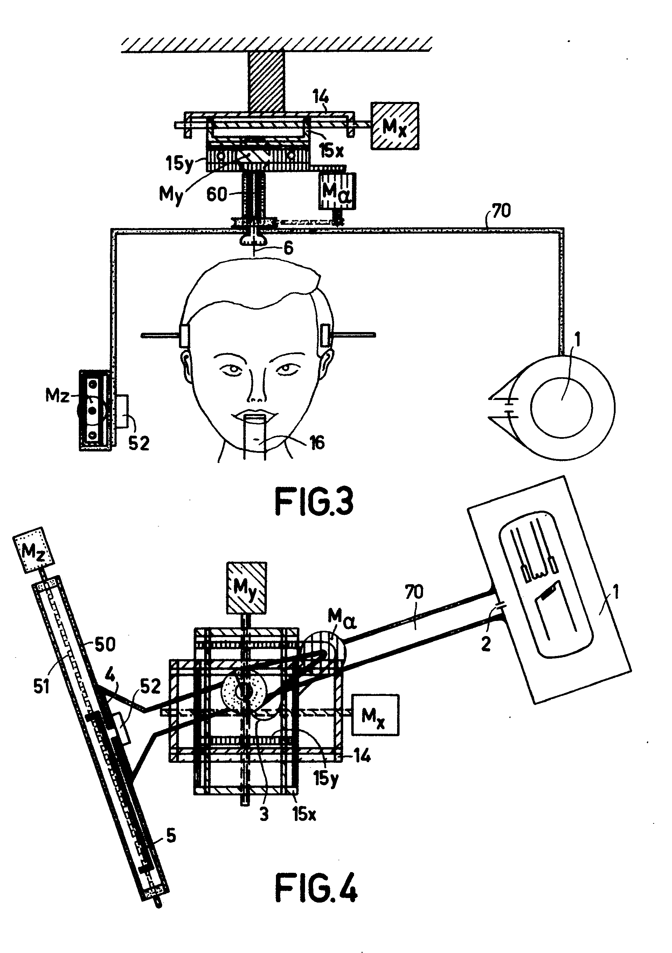

Der mechanische Aufbau eines zur Anwendung in Verbindung mit der Erfindung geeigneten Dentaltomographen ist in den Fig. 3 und 4 dargestellt. An einem ortsfesten Gestell 14, das in der Zeichnung schräg von links unten nach rechts oben schraffiert dargestellt ist, ist auf eine nicht näher dargestellte Weise ein Schrittmotor $M_x$ angebracht, der über einen Spindelantrieb den Schlitten $15_x$ in x-Richtung verschiebt. Auf dem Schlitten $15_x$ (schräg von links oben nach rechts unten schraffiert) ist ein Motor $M_y$ angebracht, durch den ein weiterer Schlitten $15_y$ (vertikal schraffiert) in y-Richtung verschiebbar ist. Der Schlitten $15_y$ trägt eine senkrecht verlaufende Achsstange 60, deren Mittelachse mit der Achse 6 identisch ist (vgl. Fig. 1). Um die Achsstange 60 ist ein Träger 70 schwenkbar, der an seinem einen Ende den Röntgenstrahler 1 und an seinem anderen Ende einen Filmhalter 50 trägt, der die Spaltblende 4 umfasst, in dem der Film 5 in einer senkrechten Ebene angeordnet ist und der durch einen weiteren Schrittmotor $M_z$ und einen Spindelantrieb 51 senkrecht zur Horizontalebene verschoben werden kann. Die Schwenkung des Trägers 70 um die Achsstange 60 bzw. deren Mittelachse 6 erfolgt durch einen Schrittmotor $M_\alpha$. Ausserdem sind zur Fixierung des Patientenkopfes verstellbare Kopfstützen 16 vorgesehen. Eine in den Mund des Patienten einführbare, in einer waagerechten Stellung im Strahlengang des Röntgenstrahlers fixierbare Beißstütze, auf die der Patient zum Fixieren beisst, sorgt dafür, dass sich die Zahnspitzen in einer definierten Ebene befinden. Eine Dosisleistungsmesseinrichtung 52, die auch hinter dem Film angeordnet sein kann, liefert ein der Intensität der Röntgenstrahlung jenseits des Kiefers des Patienten entsprechendes Signal.

Fig. 5 zeigt die Schaltung zur Steuerung der Schrittmotoren. Wie bereits im Zusammenhang mit Fig. 2a und Fig. 2b erwähnt, sind die Schaltnoppen 9b (in Fig. 5 sind nur vier dargestellt, obwohl insgesamt 30 verwendet werden) mit je einer Leitung 11b verbunden. Die Leitungen 11b führen einerseits zu den Dateneingängen eines Rechners 100 und sind andererseits über je einen Widerstand R mit einem Potential $U_1$ verbunden. Die leitfähige Unterseite der Membran 12 führt das Potential $U_0$. Wird demgemäss die Membran oberhalb einer Schaltnoppe durch das Gebiss heruntergedrückt, dann führt die zugeordnete Leitung 11b das Potential $U_0$; bleibt hingegen die Schaltnoppe unbetätigt, dann führt die zugeordnete Leitung das Potential $U_1$. Den beiden verschiedenen Potentialen $U_1$, $U_0$ kann im Rechner ein binäres Signal 1 bzw. 0 zugeordnet werden, so dass ein die Gebissform kennzeichnendes Bitmuster entsteht.

Der Rechner 100 kann ein mit Hilfe eines Mikroprozessors und der erforderlichen Speicher und Ein- und Ausgabeelemente aufgebauter Mikrocomputer sein. In ihm sind u. a. die Koordinaten der einzelnen Schaltnoppen gespeichert. Aus den Koordinaten derjenigen Schaltnoppen, die beim Zubeissen betätigt werden (deren zugeordnete Leitung 11 also das Potential $U_0$ führt), ermittelt der Rechner wie nachher näher ausgeführt wird, eine analytische Funktion y = f(x), wobei y vorzugsweise ein Polynom vierten Grades ist, so dass also gilt :

$$y = a_0 + a_1 x + a_2 x^2 + a_3 x^3 + a_4 x^4 \qquad (9)$$

Dabei hat es sich als zweckmässig erwiesen, zusätzlich auch noch die Lage der Schneidezähne durch drei Koordinatenpaare (x ; y) vorzugeben : (− 0,3 ; 0), (0 ; 0) une (0,3 ; 0). Die Koeffizienten $a_0 \ldots a_4$ werden dann so gewählt, dass die Summe der Quadrate der Abstände zwischen den durch die Schaltnoppen bestimmten und gegebenenfalls durch Mittelwertbildung berechneten koordinatenpaaren einerseits und der Kurve y andererseits minimal wird. Die mit diesem koeffizienten berechnete Funktion y stimmt im allgemeinen sehr genau mit der tatsächlichen Gebissform überein.

Wenn der Rechner 100 die Gebissform ermittelt hat, d. h. die Koeffizienten $a_0 \ldots a_4$ der Funktion y bestimmt hat, steht sogleich auch die Ableitung zur Verfügung :

$$y' = a_1 + 2a_2 x + 3a_3 x^2 + 4a_4 x^3 \qquad (10)$$

Aufgrund von Gleichung (10) kann nun auch Gleichung (8) ermittelt werden.

Das Ausgangssignal der Dosisleistungsmesseinrichtung 52, das der Dosisleistung D(t) entspricht, wird über einen Analog-Digital-Wandler 150 ebenfalls dem Rechner zugeführt, so dass dieser nun alle vom Gebiss abhängigen Parameter y, D(t) zur Berechnung der Geschwindigkeiten, dx/dt, dy/dt, w und der jeweiligen Winkelstellung $\alpha$ nach den Gleichungen (6) bis (8) und (2) erfasst hat.

Die Schrittmotoren $M_\alpha$, $M_z$, $M_x$ und $M_y$ sind an jeweils eine elektronische Motortreiberschaltung 106-109 angeschlossen, die je an einem ersten Eingang ein Richtungssignal $S_\alpha$, $S_y$, $S_x$ und $S_g$ empfängt, das die Drehrichtung der Motoren $M_\alpha$, $M_z$, $M_x$, $M_y$ bestimmt. Die Motortreiberschaltungen 106-109 empfangen jeweils an einem zweiten Eingang eine Reihe elektrischer Impulse, deren Frequenz die Drehgeschwindigkeit (Schrittfrequenz) der Schrittmotoren $M_\alpha$, $M_z$, $M_x$, $M_y$ bestimmt. Die Reihen elektrischer Impulse werden von Impulsformerstufen 102-106 generiert, even Eingang jeweils mit einem Ausgang eines Spannungs-Frequenz-Wandlers 112-115 verbunden ist. Jeder Spannungs-Frequenz-Wandler 112-115 erzeugt ein Niederfrequenzsignal, das vom Spannungspegel am Eingang des Wand-

5

**0 035 307**

lers 112-115 abhängig ist. Die analogen Spannungen an den Eingängen der Wandler 112-115 werden zusammen mit den Richtungssignalen vom Rechner 100 geliefert wie nachstehend näher beschrieben wird.

Der Rechner steuert zunächst die Motoren $M_x$ und $M_y$ in eine bestimmte Ausgangslage, wobei der Film 5 in eine Position z = 0, die Achse 60 in die Position 0 · (x = 0, y = 0) gelangt und die Gerade 7 (Fig. 1) quer zur x-Achse gerichtet ist ($\alpha = \pi/2$). Diese Ausgangslage 0 (Fig. 2) wird mittels Microschalter 9bx, 9by, 9bz, 9b$\alpha$ überwacht, die alle geschlossen sind, sobald die Achse 6 (Fig. 1) den Koordinatenursprung 0 (Fig. 2) und der Film 5 sowie die Gerade 7 ihre gewünschte Lage erreicht haben. Die Microschalter 9bx, 9by, 9bz, 9b$\alpha$ (deutlichkeitshalber nicht in Fig. 3 gezeigt) sind jeweils auf dem Gestell 14, auf dem Schlitten 15x, auf dem Filmhalter 50 und auf dem Schlitten 15g angebracht und werden jeweils vom Schlitten 15x, vom Schlitten 15y, vom Film 5 und von der Achse 60 betätigt, sobald diese in die gewünschte Position (x = 0, y = 0, z = 0, $\alpha = \pi/2$) gefahren sind. Von der Ausgangslage 0 wird das Dental-Tomographiegerät in eine Anfangsposition entlang der mit dem Rechner 100 ermittelte Kurve gefahren. Die Anfangsposition liegt an einem Endpunkt dieser Kurve, z. B. « links oben » auf der Kurve nach Gleichung (9) bei x < 0, so dass $y_M$ = 4,5 cm. Ausserdem steuert der Rechner den Schrittmotor $M_\alpha$ für die Schwenkung des Trägers gemäss Gleichung (8) so, dass der Träger senkrecht zur Kurve y verläuft, und er steuert den Schrittmotor $M_z$ so, dass die Filmkassette eine Anfangsstellung erreicht. Der Rechner 100 berechnet dann jeweils für ein Zeitinkrement $\Delta t$ (z. B. 50 ms), wieviel Schritte die vier Schrittmotoren innerhalb dieses Zeitinkrementes ausführen müssen. Allgemein gilt für das n-te Zeitinkrement :

$$\Delta x_n = c \cdot D_{n-1}/\sqrt{1 + (y'_{n-2})^2} \cdot \Delta t = x_n - x_{n-1} \tag{11}$$

Dabei ist $D_{n-1}$ die Dosis bzw. der Mittelwert der Dosisleistung während des vorhergehenden Zeitinkrementes ; $y'_{n-1}$ wird nach Gleichung (10) für $x = x_{n-1}$ berechnet. $\Delta x_n$ ist dabei die Strecke, um die der Träger bzw. seine Schwenkachse in x-Richtung verschoben werden muss. Sie ist der Schrittzahl proportional. Daraus lässt sich die Frequenz $F_{xn}$ ermitteln, die durch den Wandler 114 erzeugt werden muss, damit der Motor $M_x$ während des n-ten Zeitinkrementes $\Delta t$ den Schlitten 15x gerade um die Strecke $\Delta x_n$ verschiebt :

$$F_{xn} \approx \Delta X_n/S_x \cdot \Delta t = V_x \cdot \Delta t/S_x \cdot \Delta t = dx/dt \cdot 1/S_x \tag{12}$$

Dabei ist $F_{xn}$ die zu erzeugende Frequenz und $S_x$ die Strecke, um die der Schrittmotor $M_x$ den Schlitten 15x bei einem Schritt verschiebt.

Für die anderen Schrittmotoren gilt :

$$\Delta y_n = y_n - y_{n-1} \tag{13}$$

$y_n$ wird nach Gleichung (9) für den nach Gleichung (11) bestimmten Wert $x_n$ berechnet. $y_{n-1}$ ist bereits während des vorigen Zeitinkrementes berechnet worden. Die Schwenkung $\Delta \alpha_n$ während des n-ten Zeitinkrementes berechnet sich zu :

$$\Delta \alpha_n = \text{arc ctg}(- y'_n) - \alpha_n \tag{14}$$

$$W_n = \Delta \alpha_n/\Delta t \tag{14b}$$

$\alpha_n$ ist noch vom vorherigen Zeitinkrement bekannt. Die Verschiebung $\Delta z_n$ berechnet sich zu :

$$\Delta z_n = m \cdot \sqrt{(\Delta x_n)^2 + (\Delta y_n)^2} \tag{15}$$

Den Grössen $\Delta y_n$, $\Delta \alpha_n$ und $\Delta z_n$ lassen sich analog zur Gleichung (12) Frequenzen $F_y$, $F_\alpha$ und $F_z$ zuordnen, die mittels eines dazu proportionalen Spannungswertes $V_y$, $W_\alpha$, $V_z$ vom Rechnerausgang in den Spannungs-Frequenzwandlern 112-115 erzeugt werden. Für das erste Zeitinkrement muss in Gleichung (11) ein Schätzwert $D_0$ angesetzt werden.

Bei dieser Steuerung durch den Rechner ist die Geschwindigkeit in x-, y- und z-Richtung sowie die Winkelgeschwindigkeit der Schwenkbewegung jeweils während des Zeitinkrementes $\Delta t$ konstant und jeder der Schrittmotoren führt während eines solchen Zeitinkrementes im allgemeinen eine grössere Anzahl von Schritten (z. B. grösser 20) durch. Das heisst, dass die errechnete Kurve von einer grossen Vielzahl (200-400) von Geraden angenähert wird. (Die Gesamtdauer einer Aufnahme liegt zwischen 10 und 20 s.) In Wirklichkeit müsste sich die Geschwindigkeit aber kontinuierlich ändern, zumindest aber von Schritt zu Schritt. Der Rechner kann daher auch so programmiert sein, dass er für jeden Schrittmotor die Zeit berechnet, die bis zum nächsten Schritt (bei konstanter Schrittweite) verstreichen muss, und dass er zu dem berechneten Zeitpunkt einen Steuerimpuls direkt an den dem betreffenden Schrittmotor $M_\alpha$ ... $M_y$ vorgeschalteten Impulsformer 106 ... 109 gibt. In dem Fall kann sich also die Geschwindigkeit von

6

Schritt zu Schritt ändern, wodurch die Genauigkeit noch verbessert wird.

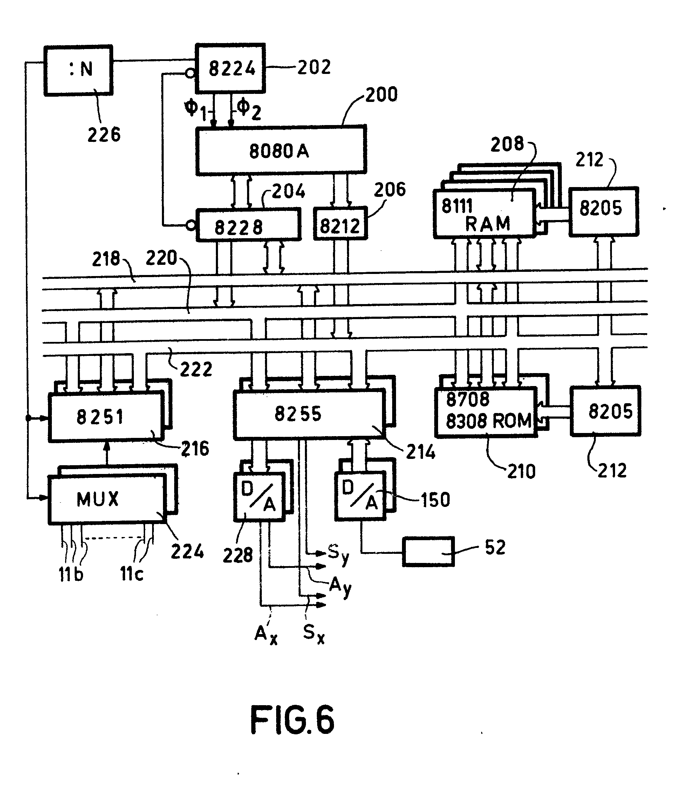

In Fig. 6 ist der in Fig. 5 dargestellte Rechner 100 detaillierter wiedergegeben. Der Rechner 100 enthält einen (Intel 8080A) Mikroprozessor 200, einen Taktgeneratortreiber 202 (Intel Typ 8224), einen bidirektionaler Bustreiber 204 (Typ 8228), einen Adresspuffer 206 (Typ 8212), einen Lese- und Schreibspeicher 208 (RAM vom Typ 8111), einen Festwertspeicher 210 (ROM Typ 8308 oder 8708), einen Adressdekodierer 212 (Typ 8205) und Ein- und Ausgabeschnittstellen 214 und 216 (vom Typ 8255 und 8251), die mittels eines Datenbusses 218, eines Steuerbusses 220 und eines Adressbusses 222 miteinander verbunden sind. Die Steuer- und Datenausgänge des Mikroprozessors 200 werden mittels der Bus-Treiberschaltung 204 mit dem Steuer- (220) bzw. Datenbus (218) verbunden. Die Adressausgänge des Mikroprozessors 200 sind mit den Eingängen des Adresspuffers 206 verbunden, deren Ausgänge mit dem Adressbus 222 verbunden sind.

Die Speicher 208 und 210 werden mit je einem Adressdekodierer 212 getrieben, damit der Mikroprozessor 200 über einen ausreichenden Speicherraum verfügen kann. Die Eingangsanschlüsse ($A_0$, $A_1$, $A_2$, $\bar{E}_1$, $\bar{E}_2$) des Adressdekodierers 212 sind an einen Teil ($A_{10} - A_{15}$) des Adressbusses ($A_0 - A_{15}$) angeschlossen. Mit den Ausgängen des Adressdekodierers 212 wird ein Teil des ganzen Speicherraumes (mittels der CS-Eingänge der integrierten Speicherschalungen) ausgewählt, von dem ein Speicherplatz mit dem restlichen Teil des Adressbuses ($A_0 - A_0$) zugänglich wird. Im (RAM) Speicher 208 werden die zur Verfügung stehenden oder errechneten variablen Daten gespeichert. Im (ROM) Festwertspeicher 210 sind die einzelnen Instruktionsbefehle oder Sequenzen gespeichert, sowie die Koeffizieten $a_1 \ldots a_4$ der unterschiedlichen Kurven.

Die Ein- und Ausgabeschnittstellen 214 und 216 enthalten eine Schaltung 214 zum Empfangen von seriellen Daten und eine Schaltung 216 zur Ein- und Ausgabe von parallelen Daten. Mit der Schaltung 214 und einer damit synchronisierten multiplexschaltung 224 (beide empfangen Synchronisationssignale, die mittels eines Frequenzteilers 226 von der Oszillatorfrequenz des Taktgenerators 202 abgeleitet werden) werden die Angaben auf den Leitungen 11b oder 11c seriell eingelesen und zwischengespeichert. Danach werden die Daten parallel auf den Datenbus 218 übertragen und weiterverarbeitet. Nachdem die Daten vom Mikroprozessor 200 zum Steuern der Motoren $M_x$, $M_y$, $M_z$, $M_\alpha$ ermittelt worden sind, werden diese Daten mittels der Ein- und Ausgabeschaltung 214 parallel ausgegeben. Die Ausgänge der Schaltung 214 werden gruppenweise je einem Digital-Analog-Wandler 228 zugeführt, wobei das Ausgangssignal $A_x$, $A_y$, $A_z$, $A_\alpha$ jedes Wandlers 228 zu der erwünschten Geschwindigkeit $V_x$, $V_y$, $V_z$, $W_\alpha$ proportional ist. Zu jedem Ausgangssignal $A_x$, usw. gehört ein Richtungssignal $S_x$, $S_y$, $S_z$, $S_\alpha$, das die Drehrichtung (links- oder rechtsum) der Motoren $M_x$, $M_y$, $M_z$, $M_\alpha$ bestimmt. Das Richtungssignal wird ohne Umwandlung von der Ausgangsschaltung 214 zu einer der Motortreiberschaltungen 106-109 geführt. Weiter wird mit der Ein- und Ausgabeschaltung 214 die von der Dosisleistungsmessvorrichtung 52 gemessene Dosisleistung D(t), die vom Analog-Digital-Wandler 150 in eine digitale Form verwandelt ist, in parallelem Bitformat über den Datenbus 218 zur Weiterverarbeitung durch den Mikroprozessor 200 übertragen.

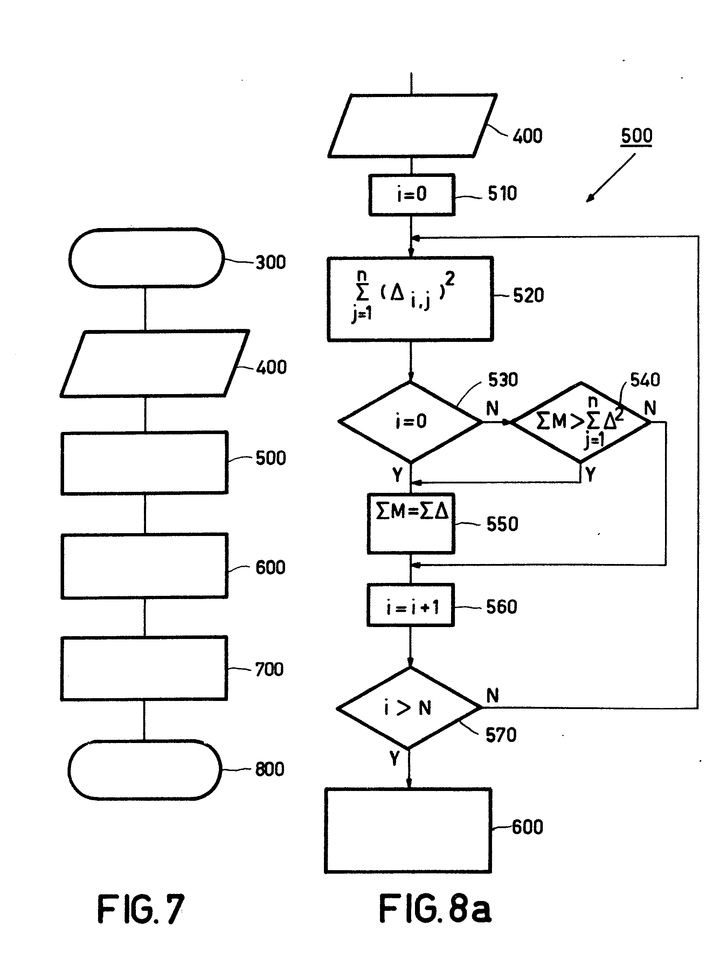

In Figur 7 ist ein Grundplan für den Programmablauf des Rechners 100 dargestellt. Nachdem das Programm gestartet ist (300), werden im ersten Programmteil (400) die Schaltzustände der Schalter 11 auf der Grundplatte 8 eingelesen und gespeichert. Während eines zweiten Programmteiles 500 wird unter Verwendung der gespeicherten Schaltzustände eine Kurve bestimmt, die sich am besten an die wirkliche Gebisskurve annähert. Im dritten Programmteil (600) wird das Dental-Tomographiegerät in eine Anfangsposition gefahren, die von der ermittelten Gebisskurve bedingt ist. Im Programmteil (700) wird die Röntgenaufnahme des Gebisses gemacht, wobei das Gerät vom Rechner 100 mittels der errechneten Daten gesteuert wird. Nach der Aufnahme wird das Gerät abgeschaltet (800).

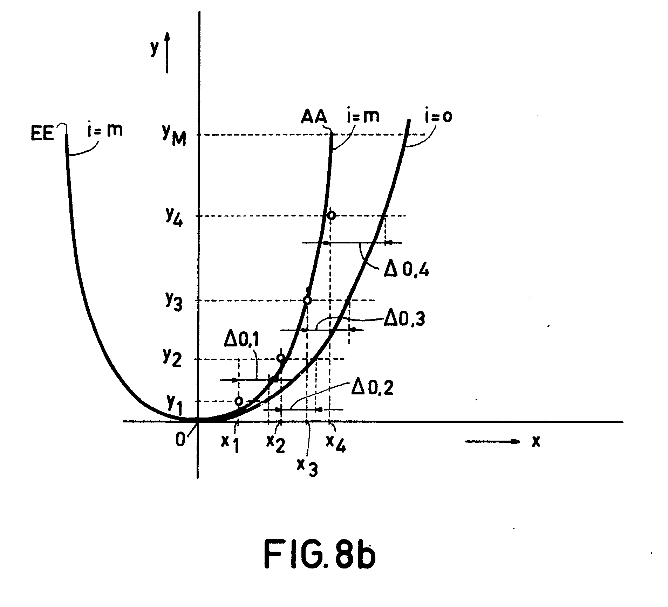

In Figur 8a ist im Detail das Flussdiagramm zur Bestimmung einer der wirklichen Gebisskurve angenäherten Kurve wiedergegeben. Dabei wird davon ausgegangen, dass eine Vielzahl N (z. B. N = 64) von unterschiedlichen Kurven im Speicher 210 gespeichert ist, das heisst dass für jede Kurve die Koeffizienten $a_{1,i} \ldots a_{4,i}$ ($0 \leq i < 64$) gespeichert sind. Weiter sind für jede Kurve diejenigen x-, y-Wertepaare gespeichert, deren y-Werte mit den jenigen der Schalter 9b auf der Grundplatte 8 übereinstimmen. Nachdem für jeden Schalter 9b geprüft worden ist (400), ob dieser Schalter geschlossen ist oder nicht, wobei der Schaltzustand gespeichert wird, werden die Koordinatwerte der geschlossenen Schalter 9b mit den jenigen Koordinaten auf einer ersten Kurve (i = 0, Schritt 510-520) verglichen, die die gleichen Koordinatenwert für die y-Werte haben. Dabei wird die Differenz $\Delta_{0,j}$ gebildet, die den Abstand in x-Richtung zwischen den Koordinaten ($x_j$ ; $y_j$) des Schalters und denen des Schnittpunktes ($x_i$ ; $y_j$) der Kurve i mit dem Koordinatengerade $y = y_j$ darstellt. In Figur 8b ist dies näher erläutert. In dem x-y Koordinatsystem sind die koordinaten ($x_1, y_1$) ... ($x_4, y_4$) von vier geschlossenen Schaltern wiedergegeben. Weiter sind im Koordinatensystem zwei Kurven (i = 0, i = m) gezeichnet. Der Abstand zwischen dem zu einem Schalter j gehörenden koordinatenpunkt ($x_j, y_j$) und dem Schnittpunkt der Kurve i = 0 mit der Gerade $y = y_j$ ist mit $\Delta_{0,j}$ angedeutet, wobei j die Werte 1 bis 4 annimmt. Der Abstand zwischen dem Punkt ($x_2, y_2$) und dem Schnittpunkt der Kurve i = 0 mit der Gerade $y_j = y_2$ ist mit $\Delta_{0,2}$ bezeichnet usw.

Aus Fig. 8a ist ersichtlich, dass im Programmschritt 520 zuerst für die Kurve i = 0 die Summe $\sum_{j=1}^{n} (\Delta_{0,j})^2$ errechnet wird, dabei ist n die Zahl der geschlossenen Schalter. Es wird darauf hingewiesen, dass sowohl links (x < 0) als auch rechts (x > 0) der y-Achse die Abstände zwischen Schalterkoordinaten

7

und Schnittpunkten errechnet werden müssen. Dabei wird der x-Wert positiv oder negativ verwendet, um die Koordinatwerte zueinander gehörender Schnittpunkte und Schalter zur Differenzbildung miteinander zu vergleichen.

Die errechnete Summe $\sum \Delta_{i\,;\,j}^2$ wird einen Minimumwert haben, indem die Kurve sich am besten an allen Schaltern 9b annähert. Wird die Summe $\sum \Delta_{i\,;\,j}^2$ zum ersten Mal errechnet (i = 0, Testschritt 530), so wird eine Prüfzahl $\sum M$ gleich $\sum \Delta_{0\,;\,j}^2$ (das bisherige Minimum) und eine Grösse « INDEX » gleich i = 0 gesetzt (Schritt 540). Die Grösse « INDEX » deutet an, welcher Satz von Koeffizienten $a_{i,1}$ ... $a_{i,4}$ zu der Kurve gehört, von der die dazugehörende Summe $\sum \Delta_{i\,;\,j}^2$ minimal ist. Der Zähler i wird dann um eins erhöht (Schritt 560) und danach wird geprüft, ob i grösser ist als N, wobei N die Zahl der gespeicherten Kurven ist. Für i = 1 wird mit dem Programmschritt 520 weitergegangen, wobei $\sum \Delta_{1\,;\,j}^2$ errechnet wird für die Kurve mit den Koeffizienten $a_{1,1}$ ... $a_{1,4}$. Da i $\neq$ 0 ist, wird jetzt geprüft, ob der neu errechnete Wert $\sum \Delta_{1\,;\,j}^2$ kleiner ist als die Prüfzahl $\sum M$. (Wenn dies nicht der Fall ist, dann wird die Prüfzahl $\sum M$ und die Grösse INDEX nicht geändert (keine bessere Annäherung ermittelt) und das Programm geht bei Schritt 560 weiter. Ist $\sum \Delta_{1\,;\,j}^2$ kleiner als $\sum M$, dann ist eine bessere Annäherung gefunden als bisher ermittelt und die Prüfzahl $\sum M$ wird gleich $\sum \Delta_{1\,;\,j}^2$ und die Grösse INDEX gleich i = 1 gemacht (Programmschritt 550).

Dieser Programmteil 500 (Schritte 510-570) wird maximal N mal durchlaufen, wobei die Prüfzahl $\sum M$ und die Grösse INDEX angepasst werden, sobald für eine Kurve der Wert $\sum \Delta_{i\,;\,j}^2$ kleiner als die Prüfzahl ist. Das Resultat besteht darin, dass die Grösse INDEX nach Ablauf des Programmteils 500 den jenigen Satz von Koeffizienten $a_{i,1}$ ... $a_{i,4}$ anzeigt, der zu der Kurve gehört, die die beste Näherung der wirklichen Gebisskurve ist.

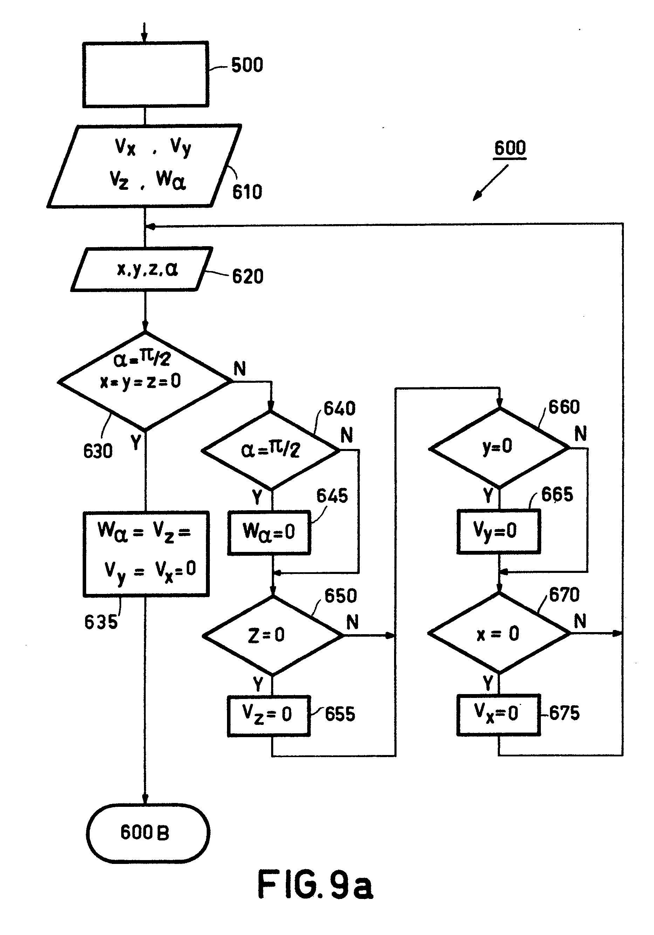

Bevor die Aufnahme gestartet wird, werden, wie vorher angedeutet, die von den Motoren $M_x$, $M_y$, $M_z$ und $M_\alpha$ angetriebenen Teile des Gerätes zuerst in eine Ausgangslage gebracht (x = 0, y = 0, z = 0, $\alpha = \pi/2$), wonach die Motoren die erwähnten Teile in eine Anfangsposition bringen, wo die Aufnahme gestartet wird. Das Bringen der Teile in die Ausgangslage ist notwendig, da das Gerät in einer von der vorherigen Aufnahme bedingten Endposition steht. Im ersten Programmschritt (610) des Programmteils 600 (Fig. 9a) werden daher die Steuersignale $V_x$, $V_y$, $V_z$ und $W_\alpha$ ausgegeben zum Steuern der Motoren $M_x$, $M_y$, $M_z$, $M_\alpha$. Im nächsten Schritt (620) werden (mittels der Mikroschalter 9bx, 9by, 9bz, 9b$\alpha$ ; Fig. 5 und 6) die Positionen x, y, z und $\alpha$ eingelesen. Sind die Bedingungen x = y = z = 0 und $\alpha = \pi/2$ erfüllt (Ausgangslage ist erreicht, alle Mikroschalter sind geschlossen), so werden die Steuersignale alle gleich Null gesetzt (635). Danach wird das Dental-Tomographiegerät nach Programmteil (600 B) in seine Anfangsposition gefahren. Sind die Bedingungen (Schritt 630) nicht erfüllt, so wird geprüft, ob die einzelnen Koordinaten ($\alpha = \pi2$, z = 0, y = 0, x = 0) erreicht worden sind (Schritte 640, 650, 660, 670). Sind eine oder mehrere Bedingungen erfüllt, so wird das eugehörende Signal ($W_\alpha$, $V_z$, $V_y$, $V_x$) gleich Null gesetzt (Schritte 645, 655, 665, 675). Danach werden die neu erreichten Koordinaten eingelesen (620), wonach deren Prüfung stattfindet usw. Die Schleife (620, 630, 640-675) wird so oft durchlaufen, bis die Bedingungen in Schritt (630) erfüllt worden sind.

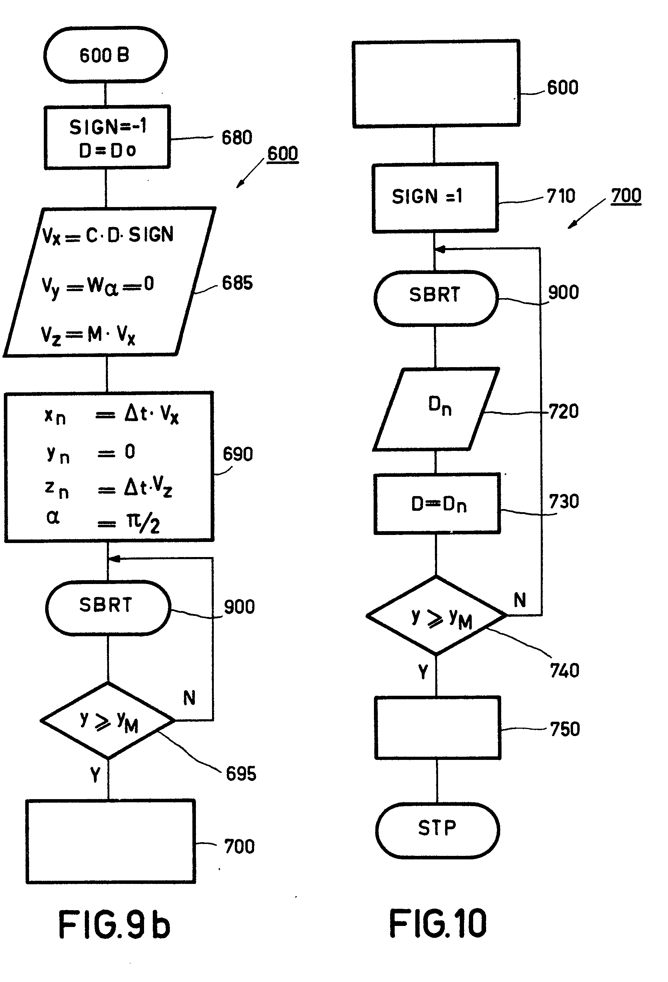

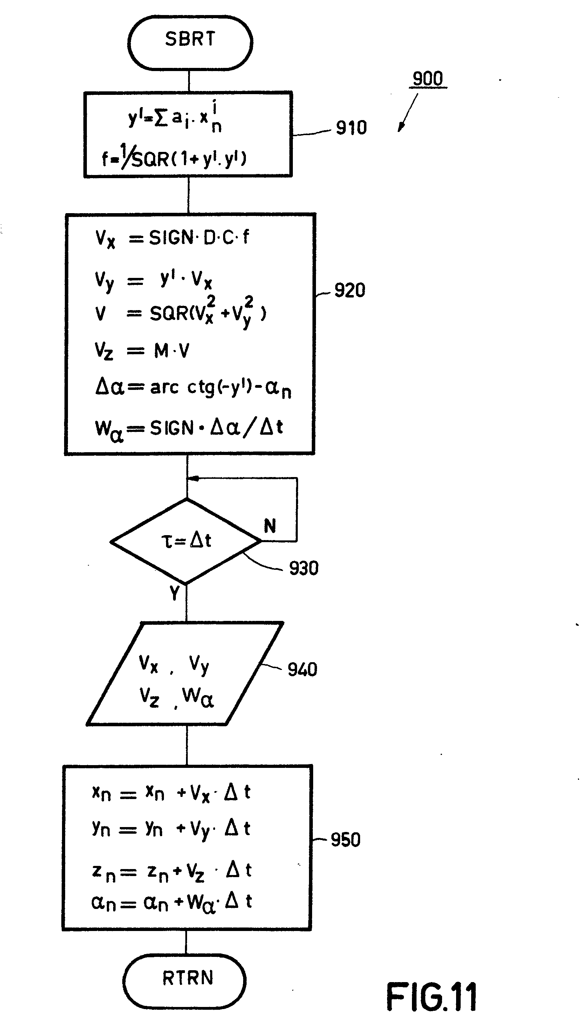

Das Tomographiegerät wird danach von der Ausgangslage in der Anfangsposition gefahren, wobei der Rechner 100 mit dem in Figur 9b gezeigten Programmteil die Motoren $M_x$, $M_y$, $M_z$, $M_\alpha$ derart steuert, dass die Anfangsposition entlang der ermittelten Kurve angefahren wird. Im Schritt 680 werden den Grössen « SIGN » und D zuerst die Werte-1 bzw. $D_0$ gegeben ($D_0$ könnte etwa der Durchschnittdosisleistung entsprechen). Die Grösse SIGN bestimmt die Drehrichtung der unterschiedlichen Motoren $M_x$, $M_y$, $M_\alpha$ und $M_z$. Die Grösse D bestimmt die Drehgeschwindigkeit dieser Motoren und hat einen festen Wert, solange keine Dosisleistung der Röntgenröhre 1 gemessen wird. Im nächsten Schritt 685 werden die Initialwerte zur Steuerung der Motoren $M_x$, $M_y$, $M_z$, $M_\alpha$ nach den Formeln (6), (4) und (2) errechnet, wobei daraufhingewiesen wird dass für die Ausgangslage die Werte y' sowie auch dy/dt = $V_y$ und dx/dt = $W_\alpha$ gleich Null sind. Danach werden die neu erreichten Koordinaten $x_n$, $y_n$, $z_n$ und $\alpha_n$ errechnet (690). Nachdem die Initialwerte der Steuerung ermittelt sind, wird eine Subroutine 900 aufgerufen zur weiteren Steuerung des Gerätes. Die Subroutine wird nachstehend mit Hilfe der Figur 11 näher beschrieben. Nach Abschliessen der Subroutine 900 wird geprüft, ob der Maximalwert $y_M$ erreicht ist. Ist dies nicht der Fall, so wird die Subroutine 900 wieder angerufen usw. Ist der Maximalwert $y_M$ erreicht, so kann mit dem Programmteil 700, der das Tomographiegerät während einer Aufnahme regelt, angefangen werden.

Der Programmteil 700 ist in Fig. 10 näher dargestellt. In erstem Schritt 710 wird die Aufnahme gestartet und der Grösse SIGN der Wert + 1 gegeben, damit die Steuersignale $V_x$, $V_y$, $V_z$, $W_\alpha$ die Motoren $M_x$, $M_y$, $M_z$, $M_\alpha$ in der entgegengesetzten Richtung steuern wie bei Programmteil 600. Danach wird die Subroutine 900 aufgerufen, die die Steuersignale $V_x$, $V_y$, $V_z$ und $W_\alpha$ sowie die neuen Koordinaten ermittelt. Im nächsten Schritt 720 wird die von der Röntgenröhre gelieferte Dosisleistung vom Rechner 100 eingelesen, damit bei den für die nächsten Steuersignale notwendigen Berechnungen in Subroutine 900 die Dosisleistung berücksichtigt werden kann. Im Schritt 730 bekommt die (bis jetzt einen festen Wert enthaltende) Grösse D den gemessenen Dosisleistungswert $D_n$. Beim Schritt 740 wird geprüft, ob der Maximumwert $y_M$ erreicht ist. Mit dem Programmteil 600 wird die Achse 6,60 (Fig. 1, 3) zum Punkt AA (Fig. 8b) gefahren. Da die Grösse SIGN von einem negativen in einen positiven Wert umgewandelt ist, wird nunmehr die Achse 6,60 zuerst vom Wert $y_M$ (Punkt AA) zu kleineren, y-Werten geführt werden. Erst nachdem der Punkt (0) passiert worden ist, steigt der y-Wert wieder an.

Sobald dieser Wert $y_M$ wieder erreicht ist (Punkt EE in Fig. 8b), ist die Aufnahmekurve ganz zurückgelegt, und die Aufnahme kann beendet werden (750). Das Programmteil 700 ist damit beendet.

Ist der Wert $y_M$ aber nicht erreicht, so wird die Subroutine 900 wieder angerufen. Die Subroutine 900 errechnet mit den vorher ermittelten Koordinaten $x_n$, $y_n$, $z_n$ und $\alpha_n$ und mit der neu gemessenen Dosisleistung $D_n$ neue Steuersignale $V_x$, $V_g$, $V_z$ und $W_\alpha$ sowie die neuen dazu gehörenden Koordinaten $x_n$, $y_n$, $z_n$, $\alpha_n$. Die Schleife 900-720-730-740 wird so oft zurückgelegt, bis die Bedingung (Schritt 740) erfüllt ist und die Aufnahme beendet werden kann.

Die Subroutine 900 ist in Figur 11 dargestellt. Im ersten Schritt (910) der Subroutine 900 wird der Wert $y'$ (abhängig von der jeweiligen Wert von $x_n$) errechnet, sowie der Wert $f = 1/\sqrt{1 + y' \cdot y'}$. Im zweiten Schritt (920) werden die Steuersignale $V_x$, $V_y$, $V_z$ und $W_\alpha$ errechnet mit Hilfe der Formeln (6), (5), (4) und (2), sowie (14) und (14b). Schritt 930 enthält eine Warteschleife. Solange das Zeitinkrement $\Delta t$ noch nicht abgelaufen ist, sollen keine neuen Steuersignale $V_x$, $V_y$, $V_z$, $W_\alpha$ den Schrittmotoren $M_x$, $M_y$, $M_\alpha$, $M_z$ bzw. deren Steuerschaltungen 102 ... 109 zugeführt werden. Die Grösse $\tau$ ist ein Zählerinhalt, der die Pulszahl enthält, die der Taktgeneratortreiber 202 seit Beginn des Zeitinkrementes $\Delta t$ erzeugt hat. Sobald $\tau$ einen Wert erreicht, der dem Ablauf des Zeitinkrementes entspricht, wird der nächste Programmschritt (940) ausgeführt. Dabei werden die neu ermittelten Steuersignale $V_x$, $V_y$, $V_z$, $W_\alpha$ den Steuerschaltungen 102 ... 115 zugeführt.

Nachdem die Steuersignale den Steuerschaltungen 102-115 zugeführt sind, werden die neuen Koordinaten $x_n$, $y_n$, $z_n$ und $\alpha_n$ errechnet. Dabei werden die vorher ermittelten Steuersignale $V_x$, $V_y$, $V_z$, $W_\alpha$ mit einbezogen ($x_n = x_n + V_x \cdot \Delta t$ usw., Programmschritt 950). Damit ist die Subroutine 900 beendet, wonach zum Programmteil 600 oder 700 zurückgesprungen wird, je nach der Stelle wo das Programm zum Ausführen der Subroutineschritte verlassen worden ist.

**Ansprüche**

1. Dental-Tomographiegerät mit einem um eine senkrechte Achse (6 ; 60) schwenkbaren Träger (70), an dem eine Strahlenquelle (1) und ein darauf ausgerichteter Filmhalter (50) befestigt sind, mit einer Verstelleinrichtung (14, 15x, 15y) zur translatorischen Verschiebung des Trägers in einer horizontalen Ebene, mit Antriebsmotoren zum Schwenken des Trägers ($M_\alpha$), zum translatorischen Verschieben des Trägers ($M_x$ ; $M_y$) und zum Verschieben eines Films ($M_z$) innerhalb des Filmhalters, und mit einer digitalen Steuereinrichtung zur Steuerung der Antriebsmotoren, dadurch gekennzeichnet, dass die digitale Steuereinrichtung einen mit beim Zubeissen betätigbaren Druckkontakten (12, 9b) versehenen, in den Patientenmund einführbaren Körper (8 ... 12) und einen Rechner (100) enthält, der aus dem Schaltzustand der einzelnen Druckkontakte (12, 9b) Steuersignale ($W_\alpha$, $V_z$, $V_x$, $V_y$) errechnet, die zur Steuerung der Antriebsmotoren ($M_\alpha$, $M_z$, $M_x$, $M_y$) für eine Verschiebung der Achse (6 ; 60) auf einer die zubeißende Gebißform und -länge annähernden Kurve dienen.

2. Dental-Tomographiegerät nach Anspruch 1, dadurch gekennzeichnet, dass der Körper eine Grundplatte (8) umfasst, auf der durch Zubeissen zusammendrückbare Kontakte (9b) angeordnet sind.

3. Dental-Tomographiegerät nach einem der vorhergehenden Ansprüche, dadurch gekennzeichnet, dass auf der Grundplatte (8) eine Vielzahl von Noppen (9a, 9b) aus einem elastischen Material mit niedrigem Elastizitätsmodul angeordnet ist, von dem ein Teil eine geringere Höhe hat als der restliche Teil und auf der von der Grundplatte abgewandten Stirnseite mit einem elektrisch leitfähigen Belag (10) versehen ist, und dass die Noppen von einer elastischen Membran (12) überspannt sind, die auf ihrer den Noppen (9a, 9b) zugewandten Seite mit einer leitfähigen Schicht versehen ist.

4. Dental-Tomographiegerät nach einem der vorhergehenden Ansprüche, dadurch gekennzeichnet, dass eine Dosisleistungsmesseinrichtung (52) vorgesehen ist, deren Ausganssignal dem Rechner (100) zwecks Steuerung der Geschwindigkeit der Antriebsmotoren ($M_\alpha$, $M_z$, $M_x$, $M_y$) entsprechend der jeweils gemessenen Dosisleistung ($D(t)$, zugeführt wird.

5. Dental-Tomographiegerät nach Anspruch 1 oder 4, dadurch gekennzeichnet, dass der Rechner (100) einen Speicher zum Speichern der Koordinaten der Druckkontakte und zum Speichern von Daten mehrerer unterschiedlicher, Gebißformen annähernder Kurven, sowie von Vergleichs- und Rechenmitteln zum Vergleichen der Koordinaten der geschlossenen Schalter mit den Kurven enthält und daß der Rechner aus einem ersten Teil von Daten für jede Kurve, einen Zahlenwert, der von den Differenzen zwischen den Koordinaten der geschlossenen Druckkontakte und dem ersten Teil der Daten jeder Kurve abhängig ist, derart berechnet, daß der Zahlenwert kleiner ist wenn die Differenzen kleiner sind, wonach der Rechner aus einem zweiten Teil der Daten, die zu derjenigen Kurve gehören, für die der kleinste Zahlenwert ermittelt worden ist, die Steuersignale ($W_\alpha$, $V_z$, $V_x$, $V_y$) für die Antriebsmotoren errechnet.

6. Dental-Tomographiegerät nach Anspruch 5, dadurch gekennzeichnet, dass jede Kurve ein Polynom höheren Grades ist, vorzugsweise vom vierten Grad, und dass der zweite Teil der im Speicher gespeicherten Daten die zu den Polynomen gehörenden Sätze von Koeffizienten sind.

7. Dental-Tomographiegerät nach Anspruch 6, dadurch gekennzeichnet, dass der erste Teil der im Speicher gespeicherte Daten diejenigen Koordinaten verschiedener Punkte jeder Kurve sind, bei denen eine der Koordinaten jedes Punktes gleich der entsprechenden Koordinate eines der Druckkontakte ist.

# 0 035 307

## Claims

1. A dental tomography apparatus, comprising a carrier (70) which is pivotable about a vertical axis (6 ; 60) and on which there are mounted a radiation source (1) and a film holder (50) which is aligned with respect thereto, an adjustment device (14, 15x, 15y) for the translatory displacement of the carrier in a horizontal plane, drive motors for the pivotal displacement of the carrier $(M_x)$, the translatory displacement of the carrier $(M_x, M_y)$ and the displacement of a film $(M_z)$ within the film holder, and also comprising a digital control device for controlling the drive motors, characterized in that the digital control device comprises an element (8 ... 12) which can be introduced into the mouth of the patient and which comprises pressure contacts (12, 9b) which can be actuated by biting, and also comprising an arithmetic unit (100) which calculates on the basis of the switching condition of the individual pressure contacts (12, 9b), control signals $(W_x, V_z, V_x, V_y)$ for controlling the drive motors $(M_x, M_z, M_x, M_y)$, for a displacement of the axis (6 ; 60) along a curve which approximates the shape and length of the relevant dentition.

2. A dental tomography apparatus as claimed in Claim 1, characterized in that the element comprises a base plate (8) on which contacts (9b) are provided which can be compressed by biting.

3. A dental tomography apparatus as claimed in any of the preceding Claims, characterized in that on the base plate (8) there are provided a plurality of studs (9a, 9b) of an elastic material having a low modulus of elastiscity, part of said studs having a height which is less than that of the other part and being provided with an electrically conductive coating (10) on that face which is remote from the base plate, an elastic diaphragm (12) being tensioned across the studs, the surface of said diaphragm which faces the studs (9a, 9b) being provided with a conductive layer.

4. A dental tomography apparatus as claimed in any of the preceding Claims, characterized in that there is provided a dose power measuring device (52) whose output signal is applied to the arithmetic unit (100) in order to control the speed of the drive motors $(M_x, M_z, M_x, M_y)$ in accordance with the dose power (D(t)) each time measured.

5. A dental tomography apparatus as claimed in Claim 1 or 4, characterized in that the arithmetic unit (100) comprises a memory for storing the coordinates of the pressure contacts and for storing data relating to serval curves approximating different dentition shapes, and also comprises comparison and arithmetic means for comparing the coordinates of the closed switches with the curves, the arithmetic unit calculating, on the basis of a first part of the data, a numerical value for each curve, which numerical value is dependent on the differences between the coordinates of the closed pressure contacts and the first part of the data relating to each curve, such that the numerical value is smaller as the differences are smaller, after which the arithmetic unit calculates the control signals $(W_x, V_z, V_x, V_y)$ for the drive motors from a second part of the data which is associated with that curve for which the smallest numerical value has been determined.

6. A dental tomography apparatus as claimed in Claim 5, characterized in that each curve is a higher degree polynomial, preferably of the fourth degree, the second part of the data stored in the memory being the sets of coefficients associated with the polynomials.

7. A dental tomography apparatus as claimed in Claim 6, characterized in that the first part of the data stored in the memory concerns these coordinates of different points on each curve, at which one of the coordinates of each point equals the corresponding coordinate of one of the pressure contacts.


## Revendications

1. Appareil de tomographie dentaire comportant un support (70) pouvant pivoter autour d'un axe vertical (6, 60), auquel sont fixés une source de rayons (1) et un porte-film (50) orienté vers cette source, un dispositif de déplacement (14, 15x, 15y) pour animer le support d'un mouvement de translation dans un plan horizontal, des moteurs d'entraînement $(M_\alpha)$ pour faire pivoter le support $(M_x, M_y)$ pour déplacer le support en un mouvement de translation et $(M_z)$ pour déplacer un film à l'intérieur du porte-film, et un dispositif de commande numérique pour commander les moteurs d'entraînement, caractérisé en ce que le dispositif de commande numérique comprend un corps (8 ... 12) pouvant être introduit dans la bouche du patient et pourvu de contacts à pression (12, 9b) pouvant être actionnés par morsure et un calculateur (100) qui, à partir de l'état de commutation des contacts à pression (12, 9b) individuels, calcule des signaux de commande $(W_\alpha, V_z, V_x, V_y)$ qui servent à commander les moteurs d'entraînement $(M_\alpha, M_z, M_x, M_y)$ pour un déplacement de l'axe (6, 60) sur une courbe se rapprochant de la forme et de la longueur de la denture déterminées par morsure.

2. Appareil de tomographie dentaire suivant la revendication 1, caractérisé en ce que le corps comporte une plaque de base (8) sur laquelle sont montés des contacts (9b) pouvant être comprimés par morsure.

3. Appareil de tomographie dentaire suivant l'une quelconque des revendications précédentes, caractérisé en ce que de nombreux tétons (9a, 9b) en une matière élastique à faible module d'élasticité sont disposés sur la plaque de base (8), en ce qu'une fraction de ces tétons a une hauteur inférieure à celle de la fraction restante et en ce qu'un recouvrement conducteur électrique (10) est prévu sur la face d'about des tétons opposée à la plaque de base une membrane élastique étant tendue au-dessus des tétons et cette membrane étant pourvue d'une couche conductrice sur son côté tournée vers les tétons (9a, 9b).

4. Appareil de tomographie dentaire suivant l'une quelconque des revendications précédentes, caractérisé en ce qu'un dispositif de dosimétrie (52) est prévu, le signal de sortie de ce dispositif étant transmis au calculateur (100) en vue de régir la vitesse des moteurs d'entraînement ($M_\alpha$, $M_z$, $M_x$, $M_y$) d'une manière correspondant au taux de dose (D(t)) chaque fois mesuré.

5. Appareil de tomographie dentaire suivant la revendication 1 ou 4, caractérisé en ce que le calculateur (100) comporte une mémoire destinée à stocker les coordonnées des contacts à pression et à stocker des données de plusieurs courbes différentes se rapprochant de formes de denture, ainsi que des moyens de comparaison et de calcul pour comparer les coordonnées des commutateurs fermés aux courbes, et en ce que le calculateur calcule à partir d'une première fraction de données, pour chaque courbe, une valeur numérique qui dépend des différences entre les coordonnées des contacts à pression fermés et de la première fraction des données de chaque courbe et ce, d'une manière telle que la valeur numérique diminue lorsque les différences sont plus petites, après quoi le calculateur calcule à partir d'une deuxième fraction des données qui appartiennent à la courbe pour laquelle la plus petite valeur numérique a été déterminée, les signaux de commande ($W_\alpha$, $V_z$, $V_x$, $V_y$) pour les moteurs d'entraînement.

6. Appareil de tomographie dentaire suivant la revendication 5, caractérisé en ce que chaque courbe est un polynôme d'ordre supérieur, de préférence du quatrième ordre, et en ce que la deuxième fraction des données stockées dans la mémoire comprend les jeux de coefficients appartenant aux polynômes.

7. Appareil de tomographie dentaire suivant la revendication 6, caractérisé en ce que la première fraction des données stockées dans la mémoire comprend les coordonnées de divers points de chaque courbe pour lesquels une des coordonnées de chaque point est égale aux coordonnées correspondantes d'un des contacts à pression.

FIG.1

FIG.2a

FIG.2b

FIG.3

FIG.4

FIG.5

FIG.6

FIG.7

FIG.8a

5

FIG. 8b

FIG. 9a

## FIG. 9 b

600 B

SIGN = -1
D = Do — 680

600

$$V_x = C \cdot D \cdot SIGN$$

$$V_y = W_\alpha = 0$$

$$V_z = M \cdot V_x$$ — 685

$$\begin{aligned} x_n &= \Delta t \cdot V_x \\ y_n &= 0 \\ z_n &= \Delta t \cdot V_z \\ \alpha &= \pi/2 \end{aligned}$$ — 690

SBRT — 900

$y \geqslant y_M$    N

Y — 695

700

## FIG. 10

600

SIGN = 1 — 710

700

SBRT — 900

$D_n$ — 720

$D = D_n$ — 730

$y \geqslant y_M$    N

740

Y

750

STP

```
                    ╭─────────╮
                   (   SBRT    )
                    ╰─────────╯
                         │
        ┌────────────────────────────────┐
        │      yᴵ = Σ aᵢ · xₙⁱ            │──── 910
        │                                │
        │    f = ½SQR(1+ yᴵ·yᴵ)          │
        └────────────────────────────────┘
                         │                        900
      ┌──────────────────────────────────┐
      │   Vₓ  = SIGN· D·C· f             │
      │                                  │
      │   V_y =  yᴵ · Vₓ                 │
      │   V   = SQR(Vₓ² + V_y² )         │──── 920
      │                                  │
      │   V_z = M·V                      │
      │                                  │
      │   Δα = arc ctg(-yᴵ) - αₙ         │
      │                                  │
      │   W_α = SIGN· Δα / Δt            │
      └──────────────────────────────────┘
                         │
                    ╱────────╲
                   ╱          ╲       N
                  (   τ = Δt    )────────┐
                   ╲          ╱          │
                    ╲────────╱──── 930
                         │ Y
                    ╱────────╲
                   ╱  Vₓ , V_y ╲──── 940
                  ╱  V_z , W_α  ╱
                  ╲────────────╱
                         │
        ┌────────────────────────────────┐
        │   xₙ = xₙ + Vₓ· Δt             │
        │                                │
        │   yₙ = yₙ + V_y· Δt            │──── 950
        │                                │
        │   zₙ = zₙ + V_z · Δt           │
        │                                │
        │   αₙ = αₙ + W_α· Δt            │
        └────────────────────────────────┘
                         │
                    ╭─────────╮
                   (   RTRN    )
                    ╰─────────╯
```

$$y^{\mathrm{I}} = \sum a_i \cdot x_n^{\,i}$$
$$f = \tfrac{1}{2}SQR(1 + y^{\mathrm{I}} \cdot y^{\mathrm{I}})$$

$$V_x = SIGN \cdot D \cdot C \cdot f$$
$$V_y = y^{\mathrm{I}} \cdot V_x$$
$$V = SQR(V_x^2 + V_y^2)$$
$$V_z = M \cdot V$$
$$\Delta\alpha = arc\ ctg(-y^{\mathrm{I}}) - \alpha_n$$
$$W_\alpha = SIGN \cdot \Delta\alpha / \Delta t$$

$$\tau = \Delta t$$

$$V_x, V_y$$
$$V_z, W_\alpha$$

$$x_n = x_n + V_x \cdot \Delta t$$
$$y_n = y_n + V_y \cdot \Delta t$$
$$z_n = z_n + V_z \cdot \Delta t$$
$$\alpha_n = \alpha_n + W_\alpha \cdot \Delta t$$

# FIG.11